# EUROPÄISCHE PATENTSCHRIFT

(11) **EP 0 560 957 B1**
(45) Veröffentlichungstag und Bekanntmachung des Hinweises auf die Patenterteilung: **29.05.1996**
(21) Anmeldenummer: 92919952.9
(22) Anmeldetag: 30.09.1992
(51) Int. Cl.: C12M 1/26, B04B 1/02, B01D 21/26, B01J 8/22

(54) **VORRICHTUNG UND VERFAHREN ZUR FÖRDERUNG UND TRENNUNG EINER SUSPENSION MIT BIOLOGISCHEN ZELLEN ODER MIKROORGANISMEN**
DEVICE AND METHOD FOR CONVEYING AND SEPARATING A SUSPENSION CONTAINING BIOLOGICAL CELLS OR MICROORGANISMS
DISPOSITIF ET PROCEDE POUR LE TRANSPORT ET LA SEPARATION D'UNE SUSPENSION CONTENANT DES CELLULES BIOLOGIQUES OU DES MICRO-ORGANISMES

(30) Priorität: 03.10.1991 CH 2924/91
(43) Veröffentlichungstag der Anmeldung: 22.09.1993
(73) Patentinhaber: B. BRAUN BIOTECH INTERNATIONAL GmbH, D-34212 Melsungen (DE)
(72) Erfinder: THALER, Thomas, CH-8006 Zürich (CH); KLEMENT, Gerhard, NL-2361 XC Warmond (NL)
(74) Vertreter: Vonnemann, Gerhard, Dr.-Ing.
(86) Internationale Anmeldenummer: CH9200197
(87) Internationale Veröffentlichungsnummer: WO9307255

(56) Entgegenhaltungen:
- EP-A- 0 224 962
- EP-A- 0 275 159
- GB-A- 1 238 401
- US-A- 3 438 890
- US-A- 3 651 945

## Beschreibung

Die Erfindung betrifft eine Vorrichtung mit einem rotierenden Trennelement und ein Verfahren zur Förderung und Trennung einer Suspension mit biologischen Zellen oder Mikroorganismen in biologischen Prozessen. Und sie betrifft auch einen Bioreaktor mit einer derartigen Förder- und Trennvorrichtung. Kontinuierliche Zellseparierung bzw. Partikelseparierung von humanen, tierischen, pflanzlichen oder hybriden Zellen, Mikoroorganismen oder von mit Zellen besetzten Trägern (Mikrocarrier) in biologischen Prozessen ist eine zentrale Aufgabe. Vor allem zur Kultivierung von empfindlichen Säugetierzellen werden entsprechend sehr hohe Anforderungen an die Zellkulturtechnologie gestellt. Dies wird erläutert z.B. im Artikel "Langzeitkultivierung von Säugetierzellen" in Technische Rundschau SULZER 3, 1990, Seite 24 bis 28, in welchem als neuester Stand der Technik auch ein Perfusionsreaktor mit rotierendem Spinfilter (MBR Spinferm Bioreaktor) beschrieben ist. Die Separierung von Zellen und Medium dient zur Rückhaltung von Zellen im Bioreaktor zwecks Erreichung hoher Zelldichten, zur kontinuierlichen Durchströmung der Zellen mit frischem Nährmedium und gleichzeitigen Entfernung von Produkt und toxischen Metaboliten und zur Vereinfachung der Aufarbeitung der von den Zellen ausgeschiedenen Wirkstoffe wie Hormone, Antikörper oder Impfstoffe, welche zellfrei abgezogen werden müssen. Die sehr empfindlichen Säugetierzellen müssen laufend in optimaler Umgebung mit ausreichender Versorgung von Nährstoffen und im speziellen mit Sauerstoff gehalten sein. Im gesamten Reaktionsbereich muss absolute Sterilität aufrechterhalten werden und die bezüglich Scherkraft sehr empfindlichen Zellen dürfen keinen hohen mechanischen Beanspruchungen ausgesetzt sein. Die beschriebenen Perfusionsreaktoren mit rotierendem Spinsieb stellen gegenüber herkömmlichen Filter-und Trennsystemen schon eine wichtige Verbesserung dar. Dennoch lagern sich auch hier am rotierenden Sieb Zellen an, was schliesslich zu dessen Verstopfung und zur Anlagerung toter, sich zersetzender Zellen führt. Mit gröberen Spinfiltern wird die Verstopfung reduziert, gleichzeitig wird jedoch die Selektivität der Trennung schlecht. Externe Zentrifugen eignen sich kaum zur Trennung von empfindlichen Zellen wegen langer Zuleitungen, eingeschränkter Sauerstoffversorgung, zu hoher mechanischer Beanspruchungen und ungenügender Sterilität bei Dichtungen der bewegten Teile.

Es ist Aufgabe der vorliegenden Erfindung, die beschriebenen Beschränkungen und Nachteile der bisherigen Zellkulturtechnologien zu überwinden und eine Vorrichtung und ein Verfahren zu schaffen, welche sich speziell zur Langzeitkultivierung von empfindlichen Säugetierzellen eignen und die eine hohe Trennwirkung ermöglichen, ohne Verstopfung der Einrichtung, und welche hohe Zelldichten und damit auch eine hohe Produktivität ermöglichen. Diese Aufgabe wird erfindungsgemäss gelöst mit einer Vorrichtung nach Anspruch 1, einem Verfahren nach Anspruch 19 und einem Bioreaktor nach Anspruch 15. Durch den rotationsspaltförmigen Durchflussraum und den Separationsabstand zwischen innerer Austrittsöffnung und äusserer Durchgangsöffnung wird auf kurzem Wege eine schonende Beschleunigung der Suspension, eine Abtrennung der Zellen und eine Zurückführung in den umgebenden Reaktionsraum erreicht. Dabei erfüllt das Trennelement gleichzeitig folgende Funktionen unter grösstmöglicher Schonung und minimaler mechanischer Beanspruchung der Zellen:
- Rasche Förderung durch die Vorrichtung
- Effiziente und selektive Trennung von Zellen und Medium
- Durchmischwirkung im Umgebungsraum.

Durch die gleichzeitige kombinierte Wirkung dieser drei Funktionen wird die gestellte Aufgabe erfüllt.

Die abhängigen Ansprüche beschreiben vorteilhafte Weiterbildungen der erfindungsgemässen Vorrichtung, des Verfahrens und des Bioreaktors. Dies sind besonders einfache Ausführungen, besonders schonende und gut optimierbare Behandlung der Zellen, Anpassbarkeit auf bestimmte Bioprozesse oder Zellarten, besonders hohe Trennwirkung oder auch besonders hohe Produktivitäten und Zelldichten. Die Erfindung wird im folgenden anhand von Beispielen und Figuren weiter erläutert. Es zeigen:
- Fig. 1: Eine erfindungsgemässe Förder- und Trennvorrichtung mit rotationsspaltförmigem Durchflussraum und separater Durchgangs - und Ausgangsöffnung;
- Fig. 2a, b: ein Beispiel mit gemeinsamer Ausgangs- und Durchgangsöffnung;
- Fig. 3a, b: ein Beispiel mit wellenförmigem Wandprofil;
- Fig. 4: ein weiteres Beispiel mit Rippen und Einbauten im Durchflussraum;
- Fig. 5a, b: Beispiele von Einlagen im Durchflussraum;
- Fig. 6: einen Bioreaktor mit Steuerungs- und Regelungseinheit;
- Fig. 7: eine Kaskade mit zwei in Serie geschalteten Trennvorrichtungen;
- Fig. 8: den zeitlichen Verlauf der Zentrifugalbeschleunigung beim Durchfliessen der Zellsuspension durch die Trennvorrichtung;
- Fig. 9: eine Separationskurve in Funktion der Zellengrösse;
- Fig. 10: Beispiele verschiedener Separationskurven und die Verschiebung der Separations-schwelle;
- Fig. 11: ein Beispiel mit Durchfluss von unten nach oben.

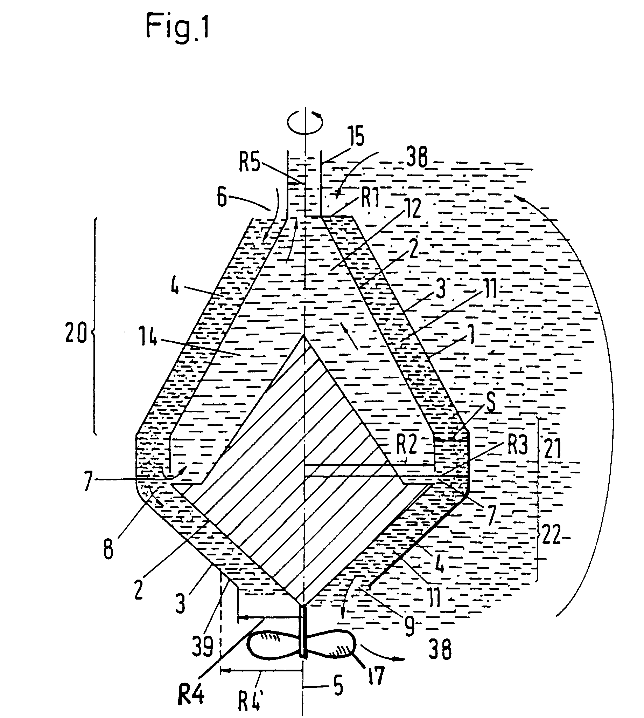

Die schematisch dargestellte Förder- und Trennvorrichtung 1 in Fig.1 weist einen rotationsspaltförmigen Durchflussraum 4 für die Zellsuspension 11 auf, welcher durch eine innere und eine äussere Begrenzungswand bzw. Begrenzungsfläche 2 und 3 gebildet ist. Aus dem umgebenden Reaktionsraum 38 fliesst die Zellsuspension 11 über eine ringförmige Eintrittsöffnung 6 mit Radius R1 in einen kegelförmigen Beschleunigungsbereich 20 des Durchflussraums 4. Das Trennelement 1 rotiert um seine Achse 5. Dabei nimmt die Zentrifugalbeschleunigung proportional zum Ringspaltradius zu bis zu einem maximalen Wert Z1 bei einem maximalen Radius R3 eines anschliessenden zylinderförmigen Separationsraums im Separationsbereich 21. Durch die Zentrifugalkräfte werden die spezifisch etwas schwereren Zellen der Suspension an die Aussenwand 3 des Durchflussraums 4 bewegt, während das leichtere zellfreie Medium 12 an der Innenwand 2 verbleibt. Am Ende des Separationsbereichs 21 wird das weitgehend zellfreie Medium 12 über eine innere Austrittsöffnung 7 mit Radius R2 abgezogen, während die Suspension 11 über die Durchgangsöffnung 8 mit Radius R3 in einen kegelförmigen Auslaufbereich 22 gefördert wird, wo die Zentrifugalbeschieunigung mit abnehmendem Radius wieder abgebaut wird, bis zur Ausgangsöffnung 9 mit Radius R4, durch welche die Suspension wieder in den Umgebungsraum 38 gelangt. Das zellfreie Medium 12 wird über einen inneren Rückflussraum 14 und eine zentrale Abführleitung 15 mit Radius R5 abgezogen.

Die unterschiedlichen Radien R2 und R3 definieren einen Separationsabstand S = R3 - R2, in dem sich die Trennung von Zellen und zellfreiem Medium ausbildet. Die dazu notwendigen Zentrifugalkräfte werden durch Wahl der Radien R und Einstellung der Drehzahl erreicht. Mit R3 wird auch die maximale Zentrifugalbeschleunigung Z1 = ω · R3 festgelegt. Der Separationsabstand S liegt vorzugsweise zwischen 0.1 und 0.3 R3. Der Ausgangsradius R4 ist grösser als der Eingangsradius R1, wodurch sich eine zentrifugale Pumpwirkung zur Förderung der Suspension im Durchflussraum 4 und zur Umwälzung im umgebenden Reaktionsraum 38 ergibt. Die Durchflussmenge der Suspension 11 liegt wesentlich höher, um mehr als eine Grössenordnung, als jene des abgezogenen Mediums 12. Entsprechend sind auch die Oeffnungen 7 und 8 dimensioniert. Mit Vorteil bilden die Querschnitte der Oeffnungen 6, 8 und 9 keine starken Verengungen, sodass keine Beschleunigungsspitzen und Turbulenzen in der Suspension auftreten. In geringerem Masse kann die Fördermenge jedoch auch noch durch diese Oeffnungsquerschnitte beeinflusst werden. Diese Oeffnungen können vorzugsweise als Ringspalt ausgebildet sein, was besonders günstige homogene Strömungsverhältnisse ergibt. Es sind aber beispielsweise auch ringförmige, grösstenteils offene Lochstreifen möglich. Die Dimension der Oeffnungen und Löcher ist jedoch immer genügend gross, um jede Verstopfungsmöglichkeit auszuschliessen und die dazwischenliegenden stege sind so schmal, dass dahinter keine toten Winkelbereiche auftreten können, in denen sich Zellen ansammeln könnten. Eine Steierung von Umwälzung und Durchfluss der Suspension 11 kann auf einfache Art erreicht werden mit einem Rührpropeller 17 in der Nähe der Ausgangsöffnung 9, welcher auf der Achse 5 des rotierenden Trennelementes 1 fixiert ist.

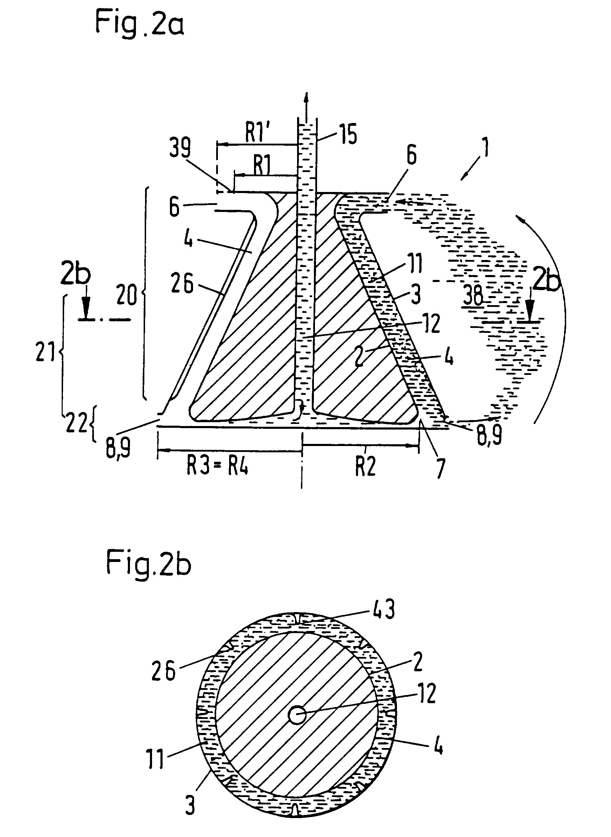

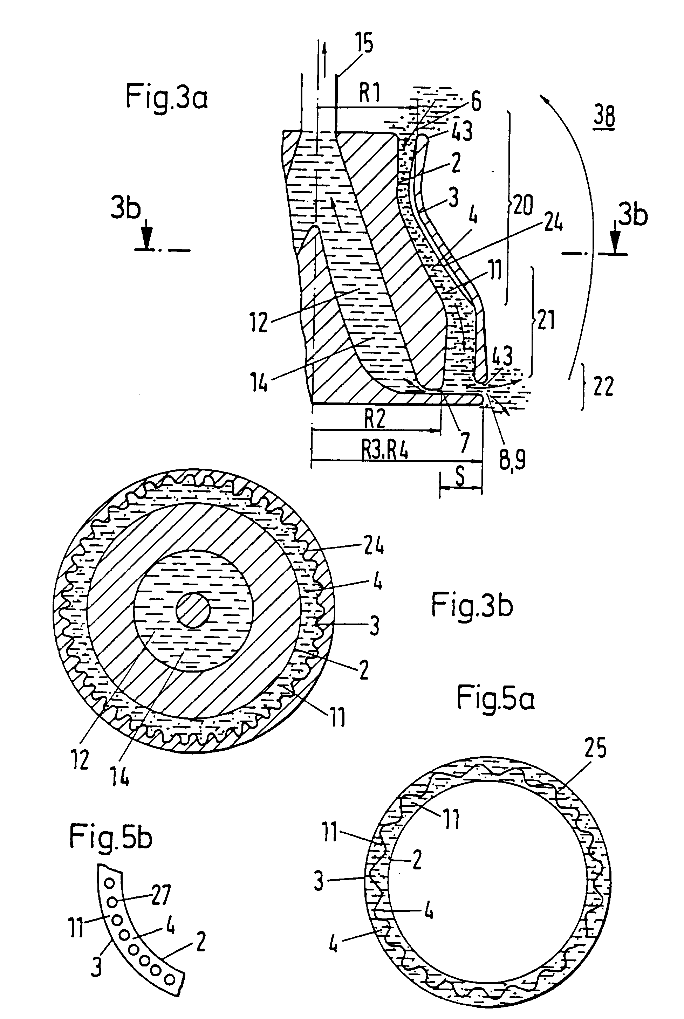

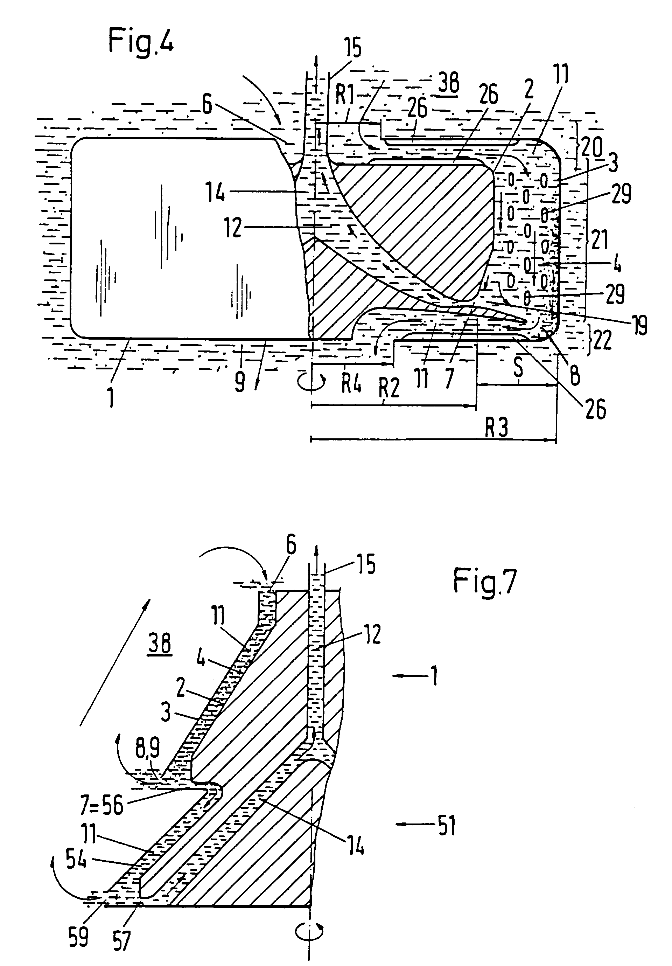

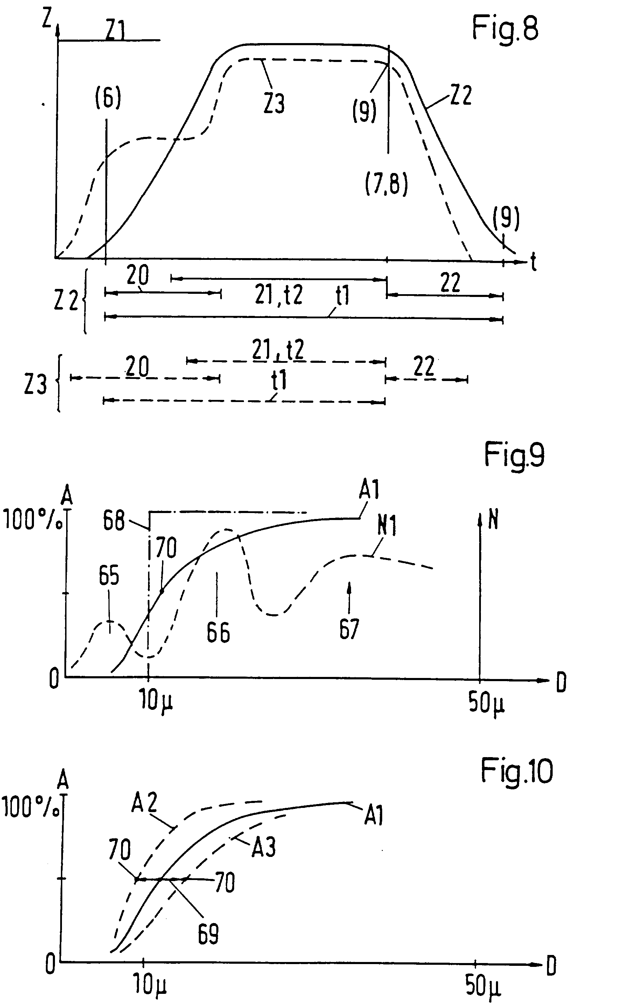

Die Figuren 2a, b zeigen ein weiteres sehr einfaches Ausführungsbeispiel, bei dem die Ausgangsöffnung 9 mit der Durchgangsöffnung 8 zusammenfällt. Damit sind auch R4 und R3 gleich gross, was eine besonders rasche Förderung der Suspension und damit kurze Durchlaufzeiten tl ergibt. Die Einstellung von gewünschtem Separationseffekt und Fördergeschwindigkeit der Suspension im Durchflussraum 4 erfolgt in erster Linie über die Drehzahl des Trennelements. Die Fördergeschwindigkeit wird vor allem durch die Radien R4 und R1 bzw. deren Differenz R4 - R1 bestimmt. Zur Erhöhung der Fördergeschwindigkeit der Suspension 11 kann z.B. gemäss Fig. 1 der Ausgangsradius R4 auf R4' vergrössert werden. Stattdessen kann aber auch gemäss Fig. 2a der Eingangsradius R1' auf R1 verkleinert werden, z.B. durch austauschbare Ringblenden 39. Wie an den Ausführungen zu Fig. 8 weiter erläutert wird, ergeben die Ausführungen gemäss Fig. 2 und 3 einerseits besonders kurze Durchlaufzeiten tl und hohe Umwäizwirkungen im Umgebungsraum 38. Andererseits treten jedoch höhere Beanspruchungen im Beschleunigungsbereich 20 und im Auslauf- oder Bremsbereich 22 auf, welche teilweise ausserhalb der Vorrichtung 1 in unmittelbarer Nähe der Oeffnungen 6 und 8 liegen.

Die Figuren 3a, b zeigen ein weiteres Beispiel mit vertikaler Eingangsöffnung 6, gemeinsamer Durchgangsöffnung 8 und Ausgangsöffnung 9 und mit einer Aussenwand 3, welche ein wellenförmiges Profil 24 aufweist. Im Beispiel von Fig. 2a, b ist eine Profilierung der Aussenwand 3 in Form von gerundeten Stegen 26 vorgesehen. Weitere Beispiele von Profilierungen im Durchflussraum 4 zeigen Fig. 5a mit einer Einlage in Form eines Wellblechs 25 und Fig. 5b mit runden Stäben 27. Im Beispiel von Fig. 4 sind sternförmige Stege 26 im Beschleunigungsbereich 20 und im Auslauf - oder Bremsbereich 22 angebracht. Im Separationsbereich 21 ist eine Ringstruktur 29 zur Homogenisierung der Suspensionsströmung 11 eingelegt. Diese Profilierungen, Einbauten und Einlagen im Durchflussraum 4 dienen dazu, eine möglichst gleichmässige Durchströmung der Suspension zu erreichen. In den Bereichen 20 und 22 soll damit eine gleichmässige, turbulenzfreie Beschleunigung bzw.

Abbremsung der Suspension erreicht werden und im Separationsbereich 21 eine möglichst gleichmässige, relativ langsame Durchströmung mit geringen Geschwindigkeitsgradienten. Vor allem im Bereich 19 vor der inneren Austrittsöffnung 7 ist die Strömungsgeschwindigkeit minimal und besonders gleichmässig. Die Form des Durchflussraums 4 ist entsprechend gestaltet. Er kann dazu aus ebenen, zylindrischen, konischen und sphärischen Abschnitten zusammengesetzt sein. Im ganzen Durchflussraum 4 der Suspension sind die Oeffnungen 6, 8 und 9 wie auch die Umlenkungen, Einbauten und Profilierungen abgerundet (43) ausgeführt, vorzugsweise mit Krümmungsradien in Strömungsrichtung von mindestens 1 mm.

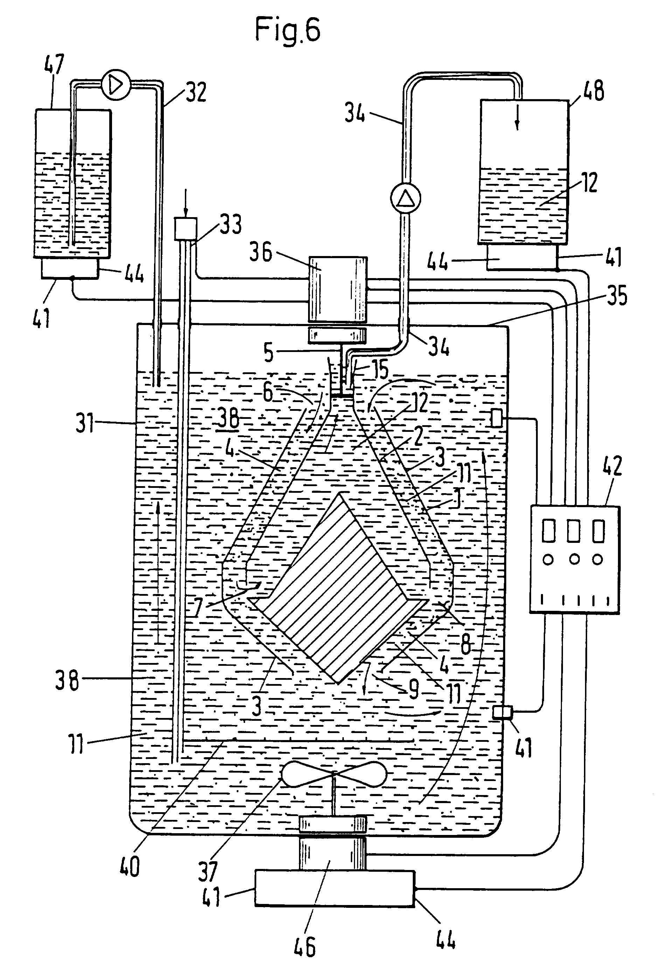

Ein erfindungsgemässer Bioreaktor in Fig. 6 weist ein Reaktionsgefäss 31 auf, welches die Förder- und Trennvorrichtung 1 und den diese umgebenden Reaktionsraum 38 einschliesst. Alle Durchführungen befinden sich hier in einem abnehmbaren Deckel 35, bei grossen Reaktoren auch in der Seitenwand. Um maximal sterile Bedingungen zu erreichen, sind nur feste und keine beweglichen Durchführen eingesetzt. Auch die Antriebsübertragung der aussenliegenden steuerbaren Motoren 36, 46 in den sterilen Reaktionsraum 38 erfolgen berührungslos, z.B. über Magnetkupplungen unter Vermeidung verschmutzender nach aussen führender Gleitlagerdichtungen. Das zellfreie Medium 12 kann berührungslos aus dem rotierenden Abflussrohr 15 in die feste Abführleitung 34 abgezogen werden. Es könnte auch eine innenliegende, steril bleibende Gleitlagerdichtung zwischen rotiertem Abflussrohr 15 und nicht bewegter Abführleitung 34 eingesetzt werden. Ueber eine Peristaltikpumpe wird das zellfreie Medium 12 dosiert in einen Erntebehälter 48 abgezogen. Von einer Vorlage 47 wird frisches Zellmedium über die Zuleitung 32 dosiert in den Reaktionsraum 38 zugeführt. Durch Gewichtssensoren 44 unter Vorlagebehälter 47, Erntebehälter 48 und Reaktionsgefäss 31 sowie durch definierte Zugabe von Betriebsstoffen 33 kann die biologische Reaktion laufend quantitativ überwacht und gesteuert werden. Dazu dienen weiter eine Steuer- und Regeleinheit 42 und damit verbundene weitere Sensoren 41 für Temperatur, Zelldichte, Konzentrationen, pH-Wert. Mit einem zusätzlichen, langsam laufenden Rührer 37 mit Antrieb 46 kann die Umwälzung im Reaktionsraum 38 unabhängig von der Pumpwirkung des Trennelements 1 noch zusätzlich gesteuert und optimiert werden. Die Versorgung mit Sauerstoff erfolgt z.B. mittels blasenfreier Begasung oder durch einen Sintersparger 40.

Fig. 7 zeigt eine Kaskade von 2 in Serie geschalteten Förder- und Trennvorrichtungen 1 und 51 mit Durchflussräumen 4 und 54, Eintrittsöffnung 6 und 56, inneren Austrittsöffnungen 7, 57 und Ausgangsöffnungen 9, 59. Die innere Austrittsöffnung 7 der ersten Trennvorrichtung bildet gleichzeitig auch die Eintrittsöffnung 56 der zweiten Trennvorrichtung. Damit lässt sich eine besonders hohe Trennwirkung erreichen.

Die Funktion der erfindungsgemässen Vorrichtung und des Verfahrens wird in den Figuren 8 bis 10 weiter illustriert. Fig. 8 zeigt den zeitlichen Verlauf der Zentrifugalbeschleunigung Z, welche auf die Zellen wirkt beim Durchlaufen der Suspension 11 durch die Trennvorrichtung. Die Kurve Z2 entspricht z.B. einer Vorrichtung gemäss Fig. 1. Im Beschleunigungsbereich 20 nach der Eintrittsöffnung 6 wird hier die Suspension möglichst gleichmässig und ohne Turbulenzen beschleunigt. Im Separationsbereich 21, welcher sich bis zu den Oeffnungen 7 und 8 erstreckt und der in der Verweilzeit t2 durchlaufen wird, erfolgt die Separation der Zellen unter relativ hoher Zentrifugalbeschleunigung nahe dem eingestellten optimalen Maximalwert Z1 von z.B. 1-2g, vorzugsweise meist nicht über 5g für empfindliche Zellen. Anschliessend folgt der Auslaufbereich 22 bis zur Ausgangsöffnung 9, in dem die Suspension wieder möglichst schonend und gleichmässig abgebremst wird. Die Durchlaufzeit tl durch die ganze Vorrichtung von der Eintrittsöffnung 6 bis zur Ausgangsöffnung 9 kann relativ kurz eingestellt werden, z.B. zwischen 10 und 100 sec, allfällig bis 300 sec. Dies ist sehr wichtig, da empfindliche Säugetierzellen nur kurze Zeit ohne Sauerstoffversorgung bestehen können und da die Nährstoffe ja erst ausserhalb der Vorrichtung 1 wieder zugeführt werden können.

Bei der Kurve Z3, welche einer Vorrichtung nach Fig. 2 oder 3 entspricht, erfolgt die Beschleunigung im Bereich 20 zum Teil schon vor der Eintrittsöffnung 6. Da hier die Oeffnungen 8 und 9 identisch sind, erfolgt auch der Abbremsvorgang im wesentlichen ausserhalb der Ausgangsöffnung 9. Die Durchlaufzeit t1 kann hier noch kürzer sein als im Beispiel Z2. Die Verweilzeit t2 im Bereich 21 kann relativ zur Durchlaufzeit t1 im Beispiel Z3 grösser sein mit entsprechend stärkerer Trennwirkung. Die Verweilzeit t2 kann hier z.B. in einem Bereich 0.5 bis 0.7 von t1 liegen. Bei der Kurve Z2 liegt die Verweilzeit t2 z.B. zwischen 0.3 und 0.5 t1. Hier sind jedoch besonders gleichmässige und schonende Beschleunigungs- und Abbremsverläufe möglich.

Fig. 9 zeigt eine Zellgrössenverteilung N1 einer Zellinie mit mittlerem Durchmesser von ca. 20 µ, d.h. die Anzahl Zellen N in Funktion ihrer Grösse D. Der unterste Bereich 65 entspricht hier toten Zellen, der mittlere Bereich 66 lebenden Einzelzellen und der obere Bereich 67 Zellaggregaten. Die Separationskurve A1 gibt den Separationsgrad in % in Funktion der Zellgrösse D an. Die Separations-schwelle 70 entspricht hier einem Separationsgrad von 50 %. Im Beispiel von Fig. 9 wird gemäss A1 also der grösste Teil der lebenden Zellen 66 und 67 von den toten Zellen 65 separiert. Eine ideale, nicht erreichbare Separationskurve 68 zum Vergleich würde z.B. einer Abtrennung aller Zellen grösser als 10µm entsprechen.

In Fig. 10 ist eine Veränderung der Separationskurve mit Verschiebung der Separationsschwelle 70 illustriert. Bei der erfindungsgemässen Vorrichtung und dem Verfahren kann die Separationskurve beeinflusst werden durch Wahl der geometrischen Parameter wie Radien R1, R2, R3, R4, Form des Durchflussraumes 4, Einbauten usw. sowie durch Variation der Betriebsparameter, d.h. vor allem der Drehzahl des Trennelements. Die optimale Einstellung erfolgt entsprechend der Art der Zellen, des biologischen Prozesses und des gewünschten Produkts. Eine Verschiebung 69 der Separationskurve nach A2 mit einer Separations-schwelle 70 bei kleineren Zelldurchmessern ergibt eine vollständigere Abtrennung der lebenden Zellen, jedoch auch mehr verbleibende tote Zellen in der Suspension 11. Umgekehrt ergibt eine Verschiebung in Richtung A3 eine vollständigere Abtrennung der toten Zellen und eine reduzierte Zurückhaltung der lebenden Zellen. Die Separationskurve kann auch während dem Betrieb verändert werden. Beispielsweise kann zum Aufbau einer Zellpopulation anfangs eine Separationskurve gemäss A2 eingestellt werden und diese mit zunehmender Zellpopulation, mittels eines Zelldichte-Sensors geregelt, kontinuierlich Richtung A3 verändert werden bis zum Erreichen einer maximalen Zelldichte in der Suspension 11. Die Zellpopulation kann aber auch periodisch abgeerntet werden. Es können lange Betriebszeiten ohne Verschlechterung der Separationseigenschaften erreicht werden.

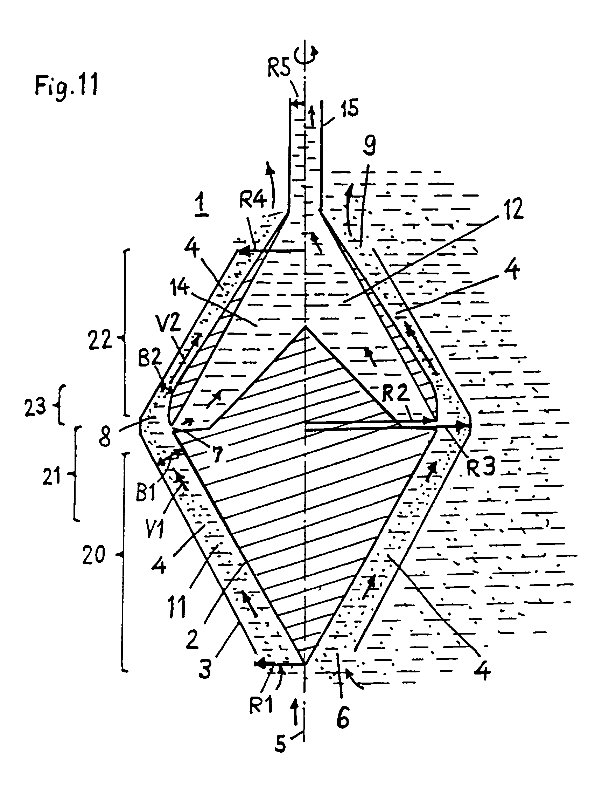

Fig. 11 zeigt eine weitere vorteilhafte Ausführung der erfindungsgemässen Förder- und Trennvorrichtung, bei der der Durchfluss von unten nach oben erfolgt. Die Suspension 11 tritt unten durch die Eintrittsöffnung 6 in den Durchflussraum 4 ein und oben durch die Austrittöffnung 9 wieder aus. Die Breite des Rotationsspaltes 4 variert dabei so, dass die Suspension im Bereich 21 langsam strömt und dort zwecks Separation lange verweilt und dass sie nach der Durchgangsöffnung 8 im Bereich 23 beschleunigt und relativ rasch abgezogen wird. Die Breite B1 des Rotationsspaltes 4 im Separationsbereich 21 ist dabei relativ gross, so dass hier eine entsprechend kleine Durchflussgeschwindigkeit V1 entsteht, während umgekehrt im Bereich 23 und 22 die Breite B2 wesentlich kleiner und die Geschwindigkeit V2 entsprechend grösser ist. B1 ist z.B. zwei bis dreimal so gross wie B2 und V2 entsprechend grösser als V1. Der ganze ringspaltförmige Durchflussraum 4 ist im übrigen so ausgebildet, dass nirgends ein Totvolumen mit unerwünscht hohen Verweilzeiten entsteht.

Ingesamt ist es möglich, entsprechend der Zellart und den gewünschten biologischen Prozessen sehr optimale Bedingungen zu realisieren, dank der schonenden Behandlung durch die erfindungsgemässe Vorrichtung und das Verfahren und dank der universellen Steuerbarkeit von Separation und Durchflussförderung. Damit kann auch die Viabilität erhöht werden, d.h. der Anteil an toten Zellen ist geringer als bei bisher bekannten Vorrichtungen.

## Patentansprüche

1. Vorrichtung zur Förderung und Trennung einer Suspension mit biologischen Zellen oder Mikroorganismen in biologischen Prozessen mit einem rotierenden Trennelement, **dadurch gekennzeichnet**, dass das Trennelement (1) eine innere (2) und eine äussere (3) Begrenzungsfläche aufweist, welche einen rotationsspaltförmigen Durchflussraum (4) einschliesst mit einer Eintrittsöffnung (6), mit einer inneren Austrittsöffnung (7) für das weitgehend zellfreie Medium (12), einer äusseren Durchgangsöffnung (8) und einer Ausgangsöffnung (9) für die Suspension (11), wobei der Abstand R4 der Ausgangsöffnung (9) von der Rotationsachse (5) grösser ist als der Abstand R1 der Eintrittsöffnung (6) und wobei der Abstand R3 der Durchgangsöffnung (8) grösser ist als der Abstand R2 der inneren Austrittsöffnung (7) von der Rotationsachse.

2. Vorrichtung nach Anspruch 1, dadurch gekennzeichnet, dass der Durchflussraum (4) einen Beschleunigungsbereich (20), darauffolgend einen Separationsbereich (21) und einen Auslaufbereich (22) aufweist.

3. Vorrichtung nach einem der vorangehenden Ansprüche, dadurch gekennzeichnet, dass der Abstand R3 der Durchgangsöffnung (8) grösser ist als der Abstand R4 der Ausgangsöffnung (9) von der Rotationsachse (5).

4. Vorrichtung nach einem der vorangehenden Ansprüche, dadurch gekennzeichnet, dass der Abstand R3 der Durchgangsöffnung (8) mindestens doppelt so gross ist wie der Abstand R1 der Eintrittsöffnung (6).

5. Vorrichtung nach Anspruch 1, dadurch gekennzeichnet, dass die Ausgangsöffnung (9) mit der Durchgangsöffnung (8) identisch ist.

6. Vorrichtung nach einem der vorangehenden Ansprüche, dadurch gekennzeichnet, dass die Eintrittsöffnung (6) konzentrisch um eine zentrale Abführleitung (15) herum angeordnet ist.

7. Vorrichtung nach einem der vorangehenden Ansprüche, dadurch gekennzeichnet, dass die Durchgangsöffnung (8) im wesentlichen ringspaltförmig ausgebildet ist.

8. Vorrichtung nach einem der vorangehenden Ansprüche, dadurch gekennzeichnet, dass der Separationsabstand S = R3 - R2 eine Grösse von 0.1 bis 0.3 R3 aufweist.

9. Vorrichtung nach einem der vorangehenden Ansprüche, dadurch gekennzeichnet, dass die innere und/oder die äussere Mantelfläche (2, 3) kegelförmige (16), zylindrische (17), ebene (18) und/oder sphärisch gewölbte Teilstücke (19) aufweisen.

10. Vorrichtung nach einem der vorangehenden Ansprüche, dadurch gekennzeichnet, dass im Durchflussraum (4) Profilierungen (24), Einbauten (25) oder Unterteilungen vorhanden sind.

11. Vorrichtung nach Anspruch 10, gekennzeichnet durch wellenförmige Profilierungen der Wände (2, 3) des Durchflussraums.

12. Vorrichtung nach Anspruch 10, dadurch gekennzeichnet, dass Rippen (26), Stäbe (27) oder Ringe (29) im Durchflussraum (4) vorhanden sind.

13. Vorrichtung nach einem der vorangehenden Ansprüche, dadurch gekennzeichnet, dass die Begrenzungen der Oeffnungen (7, 8, 9) und allfälliger Profilierungen (24) in Strömungsrichtung abgerundet sind mit einem Krümmungsradius, welcher mindestens einen Millimeter beträgt.

14. Vorrichtung nach einem der vorangehenden Ansprüche, dadurch gekennzeichnet, dass der Durchfluss im Ringspalt (4) von unten nach oben erfolgt.

15. Bioreaktor mit einer Förder- und Trennvorrichtung nach einem der vorangehenden Ansprüche mit einem einen Reaktionsraum (38) einschliessenden Reaktionsgefäss (31) für die Suspension, wobei die Eintrittsöffnung (6) und die Ausgangsöffnung (9) in den Reaktionsraum (38) führen und mit Zuleitungen für frisches Medium (32) und weitere Betriebsstoffe (33), mit einer Ableitung (34) für zellfreies Medium, sowie mit einem Antrieb (36) des rotierenden Trennelements (1).

16. Bioreaktor nach Anspruch 15 mit einem zusätzlichen, steuerbaren Rührer (37) im Reaktionsraum (38) ausserhalb der Trennvorrichtung (1).

17. Bioreaktor nach Anspruch 15 oder 16, dadurch gekennzeichnet, dass mindestens zwei Förder- und Trennvorrichtungen (1) gemäss Ansprüchen 1 bis 13 als Kaskade in Serie geschaltet sind.

18. Bioreaktor nach einem der Ansprüche 15 bis 17, gekennzeichnet durch Sensoren (41) z.B. für Temperaturen, Stoffkonzentrationen, Gewicht (44), Zelldichte und/oder Füllstand und eine mit den Sensoren und dem Antrieb (36) verbundene Steuer/Regeleinheit (42).

19. Verfahren zur Förderung und Trennung einer Suspension mit biologischen Zellen oder Mikroorganismen in biologischen Prozessen in einer Vorrichtung gemäß einem der Ansprüche 1-14, mit einem rotierenden Trennelement (1) in einem umgebenden Reaktionsraum, dadurch gekennzeichnet, dass die Suspension (11) aus dem Reaktionsraum (38) durch eine Eintrittsöffnung (6) in einen rotierenden Durchflussraum (4) gefördert und in einem Beschleunigungsbereich (20) auf eine vorgebbare Zentrifugalbeschleunigung Z1 beschleunigt wird, anschliessend während einer vorgebbaren Verweilzeit t2 einen Separationsbereich (21) durchläuft, in welchem weitgehend zellfreies Medium (12) von der Suspension (11) abgetrennt und über eine innere Austrittsöffnung (7) abgeführt wird, während die restliche Suspension über eine äussere Durchgangsöffnung (8) und/oder Ausgangsöffnung (9) in den Reaktionsraum (38) zurückgeführt wird, von wo die Suspension (11) im Kreislauf wieder in die Eintrittsöffnung (6) geführt wird.

20. Verfahren nach Anspruch 19, dadurch gekennzeichnet, dass die maximale Zentrifugalbeschleunigung Z1 zwischen 1g und 5g liegt.

21. Verfahren nach Anspruch 19 oder 20, dadurch gekennzeichnet, dass die Durchlaufzeit t1 für die Suspension von der Eintrittsöffnung (6) bis zur Ausgangsöffnung (9) zwischen 10 sec. und 300 sec. beträgt.

22. Verfahren nach einem der Ansprüche 19 bis 21, dadurch gekennzeichnet, dass die Verweilzeit t2 im Separationsbereich (21) einen Bruchteil von 0.3 bis 0.7 der Durchlaufzeit t1 beträgt.

23. Verfahren nach einem der Ansprüche 19 bis 22, dadurch gekennzeichnet, dass die Zelldichte im Reaktionsraum (38) auf vorgebbare Werte geregelt wird durch Erfassung der Zelldichte mit Hilfe eines Sensors (41) und Regelung der Drehzahl des rotierenden Trennelements (1).

24. Verfahren nach einem der Ansprüche 19 bis 23, dadurch gekennzeichnet, dass die Separationsschwelle (70) im kontinuierlichen Betrieb durch Steuerung der Drehzahl des rotierenden Trennelements (1) verändert wird.

## Claims

1. Device for conveying and separating a suspension containing biological cells or microorganisms in biological processes with a rotating separating element, characterized in that the separating element (1) comprises an internal limiting face (2) and an external limiting face (3) and encloses a flow chamber (4) in the form of a rotational gap with an inlet orifice (6), with an internal exit orifice (7) for the substantially cell-free medium (12), an external through-orifice (8) and an outlet orifice (9) for the suspension (11), the distance R4 of the outlet orifice (9) from the axis of rotation (5) being greater than the distance R1 of the inlet orifice (6) and the distance R3 of the through-orifice (8) being greater than the distance R2 of the internal exit orifice (7) from the axis of rotation.

2. Device according to claim 1, characterized in that the flow chamber (4) comprises an acceleration region (20), then a separation region (21) and a discharge region (22).

3. Device according to one of the preceding claims, characterized in that the distance R3 of the through-orifice (8) is greater than the distance R4 of the outlet orifice (9) from the axis of rotation (5).

4. Device according to one of the preceding claims, characterized in that the distance R3 of the through-orifice (8) is at least twice as great as the distance R1 of the inlet orifice (6).

5. Device according to claim 1, characterized in that the outlet orifice (9) is identical to the through-orifice (8).

6. Device according to one of the preceding claims, characterized in that the inlet orifice (6) is arranged concentrically round a central discharge pipe (15).

7. Device according to one of the preceding claims, characterized in that the through-orifice (8) is designed substantially in the form of an annular gap.

8. Device according to one of the preceding claims, characterized in that the separation distance S = R3 - R2 has a value of 0.1 to 0.3 R3.

9. Device according to one of the preceding claims, characterized in that the internal and/or external surface (2, 3) have conical (16), cylindrical (17), plane (18) and/or spherically curved portions (19).

10. Device according to one of the preceding claims, characterized in that profiling (24), fittings (25) or subdivisions are provided in the flow chamber (4).

11. Device according to claim 10, characterized by waveshaped profiling of the walls (2, 3) of the flow chamber (4).

12. Device according to claim 10, characterized in that ribs (26), rods (27) or rings (29) are provided in the flow chamber (4).

13. Device according to one of the preceding claims, characterized in that the limits of the orifices (7, 8, 9) and any profiling (24) are rounded in the flow direction with a radius of curvature of at least one millimetre.

14. Device according to one of the preceding claims, characterized in that the flow through the annular gap (4) takes place from bottom to top.

15. Bioreactor with a conveying and separating device according to one of the preceding claims with a reaction vessel (31) for the suspension enclosing a reaction chamber (38), the inlet orifice (6) and the outlet orifice (9) leading into the reaction chamber (38) and with supply pipes for fresh medium (32) and other working substances (33), with an outlet pipe (34) for cell-free medium, and with a drive (36) for the rotating separating element (1).

16. Bioreactor according to claim 15, with an additional controllable stirrer (37) in the reaction chamber (38) outside the separating device (1).

17. Bioreactor according to claim 15 or 16, characterized in that at least two conveying and separating devices (1) according to claims 1 to 13 are connected in series as a cascade.

18. Bioreactor according to one of claims 15 to 17, characterized by sensors (41), for example for temperatures, substance concentrations, weight (44), cell density and/or fullness and a closed/open loop control unit (42) connected to the sensors and the drive (36).

19. Method of conveying and separating a suspension with biological cells or microorganisms in biological processes in a device according to one of claims 1 to 14 with a rotating separating element (1) in a surrounding reaction chamber, characterized in that the suspension (11) is conveyed from the reaction chamber (38) through an inlet orifice (6) into a rotating flow chamber (4) and is accelerated in an acceleration region (20) to a predeterminable centrifugal acceleration Z1, then passes through a separation region (21) for a predeterminable residence time t2 in which substantially cell-free medium (12) is separated from the suspension (11) and discharged via an internal exit orifice (7) while the remaining suspension is returned via an external through-orifice (8) and/or outlet orifice (9) into the reaction chamber (38) from where the suspension (11) is recirculated into the inlet orifice (6).

20. Method according to claim 19, characterized in that the maximum centrifugal acceleration Z1 lies between 1g and 5g.

21. Method according to claim 19 or 20, characterized in that the passage time tl for the suspension from the inlet orifice (6) to the outlet orifice (9) is between 10 sec and 300 sec.

22. Method according to one of claims 19 to 21, characterized in that the residence time t2 in the separation region (21) is a fraction of 0.3 to 0.7 of the passage time t1.

23. Method according to one of claims 19 to 22, characterized in that the cell density in the reaction chamber (38) is adjusted to predeterminable values by detection of the cell density by means of a sensor (41) and closed loop control of the speed of rotation of the rotating separating element (1).

24. Method according to one of claims 19 to 23, characterized in that the separation threshold (70) is changed in continuous operation by open loop control of the speed of rotation of the rotating separating element (1).

## Revendications

1. Dispositif de transport et de séparation d'une suspension avec des cellules biologiques ou des micro-organismes dans des processus biologiques avec un élément de séparation tournant, caractérisé en ce que l'élément de séparation (1) possède une périphérie interne (2) et une périphérie externe (3) qui incluent un espace de passage de flux (4) en forme de fente de rotation avec une ouverture d'entrée (6) avec une ouverture de sortie (7) interne pour le milieu (12) largement dépourvu de cellules, une ouverture de passage externe (8) et une ouverture de sortie (9) pour la suspension (11), dispositif dans lequel l'espacement R4 de l'ouverture de sortie (9) à partir de l'axe de rotation (5) est plus grand que l'espacement R1 de l'ouverture d'entrée (6) et dans lequel l'espacement R3 de l'ouverture de passage (8) est plus grand que l'espacement R2 de l'ouverture de sortie interne (7) à partir de l'axe de rotation.

2. Dispositif selon la revendication 1, caractérisé en ce que l'espace de flux (4) possède une zone de ralentissement (20) suivant une zone de séparation (21) et une zone de décharge (22).

3. Dispositif selon l'une des revendications précédentes, caractérisé en ce que l'espacement R3 de l'ouverture de passage (8) est plus grand que l'espacement R4 de l'ouverture de sortie (9) à partir de l'axe de rotation (5).

4. Dispositif selon l'une des revendications précédentes, caractérisé en ce que l'espacement R3 de l'ouverture de passage (8) est au moins deux fois aussi grand que l'espacement R1 de l'ouverture d'entrée (6).

5. Dispositif selon la revendication 1, caractérisé en ce que l'ouverture de sortie (9) est identique à l'ouverture de passage (8).

6. Dispositif selon l'une des revendications précédentes, caractérisé en ce que l'ouverture d'entrée (6) est disposée d'une manière concentrique autour d'une conduite d'évacuation centrale (15).

7. Dispositif selon l'une des revendications précédentes, caractérisé en ce que l'ouverture de passage (8) est façonnée essentiellement sous forme de fente annulaire.

8. Dispositif selon l'une des revendications précédentes, caractérisé en ce que l'intervalle de séparation S = R3-R2 possède une taille de 0,1 à 0,3 R3.

9. Dispositif selon l'une des revendications précédentes, caractérisé en ce que la surface de l'enveloppe interne et/ou la surface de l'enveloppe externe (2, 3) possèdent des tronçons cintrés sphériquement (19) et/ou coniques (16), cylindriques (17) et plans (18).

10. Dispositif selon l'une des revendications précédentes, caractérisé en ce que dans l'espace de passage de flux (4), des profilages (24), des chicanes (25) ou des cloisonnements sont présents.

11. Dispositif selon la revendication 10, caractérisé par des profilages ondulés des parois (2, 3) de l'espace de passage de flux.

12. Dispositif selon la revendication 10, caractérisé en ce que des nervures (26), des barres (27) ou des anneaux (29) sont présents dans l'espace de passage de flux (4).

13. Dispositif selon l'une des revendications précédentes, caractérisé en ce que les limitations des ouvertures (7, 8, 9) et des profilages éventuels (24) sont arrondis dans la direction du courant avec un rayon de courbure qui s'élève au moins à un millimètre.

14. Dispositif selon l'une des revendications précédentes, caractérisé en ce que le flux s'effectue dans la fente annulaire (4) du bas vers le haut.

15. Bioréacteur avec un dispositif de transport et de séparation selon l'une des revendications précédentes, avec un réservoir réactionnel (31) entourant une chambre de réaction (38) pour la suspension, dans lequel l'ouverture d'entrée (6) et l'ouverture de sortie (9) conduisent dans la chambre de réaction (38) et avec des conduites d'amenée pour le milieu frais (32) et pour d'autres produits opérationnels (33), avec une dérivation (34) pour milieu dépourvu de cellule ainsi qu'avec une transmission (36) de l'élément de séparation tournant (1).

16. Bioréacteur selon la revendication 15 avec un agitateur (37) supplémentaire, manoeuvrable dans la chambre de réaction (38), en dehors du dispositif de séparation (1).

17. Bioréacteur selon la revendication 15 ou 16, caractérisé en ce qu'au moins deux dispositifs de transport et de séparation (1) selon les revendications 1 à 13 sont connectés sous forme de cascade en série.

18. Bioréacteur selon l'une des revendications 15 à 17, caractérisé par des déflecteurs (41) par exemple pour la température, la concentration en substances, le poids (44), la densité de cellules et/ou le niveau et par un élément de commande/réglage relié aux détecteurs et à la transmission (36).

19. Procédé de transport et de séparation d'une suspension avec des cellules biologiques ou des micro-organismes dans des processus biologiques dans un dispositif conforme à l'une des revendications 1 à 14, pourvu d'un élément de séparation (1) tournant dans une chambre de réaction qui l'entoure, caractérisé en ce que la suspension (11) est transportée de la chambre de réaction (38) par une ouverture d'entrée (6) dans un espace de passage de flux (4) tournant et est accélérée dans une zone d'accélération (20) à une accélération centrifuge prédéfinie Z1, ensuite pendant un temps de séjour prédéfini t2 elle traverse une zone de séparation (21) dans laquelle le milieu largement dépourvu de cellules (12) est séparé de la suspension (11) et est évacué par une ouverture de sortie (7) alors que la suspension restante est ramenée par une ouverture de passage (8) extérieure et/ou une ouverture de sortie (9) dans la chambre de réaction (38), à partir de laquelle la suspension (11) est mise à nouveau en circulation dans l'ouverture d'entrée (6).

20. Procédé selon la revendication 19, caractérisé en ce que l'accélération centrifuge maximale Z1 se situe entre 1g et 5g.

21. Procédé selon la revendication 19 ou 20, caractérisé en ce que le temps de passage tl pour la suspension se situe de l'ouverture d'entrée (6) jusqu'à l'ouverture de sortie (9) entre 10 sec. et 30 sec.

22. Procédé selon l'une des revendications 19 à 21, caractérisé en ce que le temps de séjour t2 dans la zone de séparation (21) s'élève à une fraction de 0,3 à 0,7 du temps de passage t1.

23. Procédé selon l'une des revendications 19 à 22, caractérisé en ce que la densité des cellules dans la chambre de réaction (38) est réglée à des valeurs prédéfinies par détermination de la densité de cellules à l'aide d'un détecteur (41) et réglage du nombre de tours de l'élément de séparation tournant (1).

24. Procédé selon l'une des revendications 19 à 23, caractérisé en ce que le seuil de séparation (70) en fonctionnement continu est modifié par commande du nombre de tours de l'élément de séparation tournant (1).
